# FASCICULE DE BREVET EUROPEEN

(11) **EP 1 450 834 B1**
(45) Date de publication et mention de la délivrance du brevet: **21.12.2005**
(21) Numéro de dépôt: 02793180.7
(22) Date de dépôt: 22.10.2002
(51) Int. Cl.: A61K 35/78, A61P 9/00, A61P 17/00, A61K 7/48, C11B 9/02

(54) **ABSOLUES DE BULBES D'ALLIUM SATIVUM ET UTILISATIONS A TITRE THERAPEUTIQUE OU COSMETIQUE**
ABSOLUTE ÖLE AUS ALLIUM SATIVUM UND THERAPEUTISCHE ODER KOSMETISCHE ANWENDUNGEN
ALLIUM SATIVUM BULB ABSOLUTES AND THERAPEUTIC OR COSMETIC USES

(30) Priorité: 26.10.2001 FR 0113839
(43) Date de publication de la demande: 01.09.2004
(73) Titulaire: Pierre Fabre Dermo-Cosmetique, 92100 Boulogne (FR)
(72) Inventeur: TOMATIS, Isabelle, F-31170 Tournefeuille (FR)
(74) Mandataire: Cabinet BARRE LAFORGUE & associés
(86) Numéro de dépôt international: PCT/FR2002/003618
(87) Numéro de publication internationale: WO 2003/035085

(56) Documents cités:
- EP-A- 0 464 521
- WO-A-02/36093
- DATABASE WPI Section Ch, Week 198612 Derwent Publications Ltd., London, GB; Class D21, AN 1986-078768 XP002206274 & JP 61 027910 A (KAO CORP), 7 février 1986 (1986-02-07)
- DATABASE BIOSIS [en ligne] BIOSCIENCES INFORMATION SERVICE, PHILADELPHIA, PA, US; avril 1998 (1998-04) BORDIA A ET AL: "Effect of garlic (Allium sativum) on blood lipids, blood sugar, fibrinogen and fibrinolytic activity in patients with coronary artery disease." Database accession no. PREV199800315438 XP002206271 & PROSTAGLANDINS LEUKOTRIENES AND ESSENTIAL FATTY ACIDS, vol. 58, no. 4, avril 1998 (1998-04), pages 257-263, ISSN: 0952-3278
- DATABASE FSTA [en ligne] INTERNATIONAL FOOD INFORMATION SERVICE (IFIS), FRANFURT/MAIN, DE; HWANG S L ET AL: "Studies on processing techniques of garlic (Allium sativum, Linn.)." Database accession no. 83-2-09-t0517 XP002206272 & RESEARCH BULLETIN OF TAINAN DISTRICT AGRICULTURAL IMPROVEMENT STATION 1982 HORT. SECT., TAINAN DISTRICT AGRIC. IMPROVEMENT STA., TAINAN CITY, TAIWAN,
- DATABASE WPI Section Ch, Week 197618 Derwent Publications Ltd., London, GB; Class C04, AN 1976-33191X XP002206275 & JP 51 033066 A (MITSUI Y), 19 mars 1976 (1976-03-19) cité dans la demande
- DATABASE BIOSIS [en ligne] BIOSCIENCES INFORMATION SERVICE, PHILADELPHIA, PA, US; 7 mars 2001 (2001-03-07) MOUSA AHMED S: "Discovery of angiogenesis inhibition by garlic ingredients: Potential anti-cancer benefits." Database accession no. PREV200100255676 XP002206273 & FASEB JOURNAL, vol. 15, no. 4, 7 mars 2001 (2001-03-07), page A117 Annual Meeting of the Federation of American Societies for Experimental Biology on Experimental Biology 2001;Orlando, Florida, USA; March 31-April 04, 2001 ISSN: 0892-6638

## Description

L'invention concerne des extraits de bulbes d'Allium sativum (ail) ainsi que leurs utilisations. Lesdites compositions peuvent être appliquées avantageusement et plus particulièrement dans l'industrie pharmaceutique et cosmétique. L'invention concerne donc aussi des compositions thérapeutiques (médicaments), notamment pour le traitement de l'obésité. Egalement l'invention s'étend à des compositions cosmétiques, notamment des compositions cosmétiques à application topique pour la beauté (produits de beauté) ou le soin de la peau, et des compositions cosmétiques pour le traitement préventif ou curatif par voie topique de la cellulite et/ou des surcharges adipeuses dermiques localisées.

Dans tout le texte, les termes définissant les produits d'extraction (huile essentielle, concrète, oléorésine ou résinoïde, absolue...) sont utilisés selon la terminologie définie par la norme NF T 75-006 (février 1998).

L'Allium sativum, c'est-à-dire l'ail, est depuis longtemps réputé pour sa richesse nutritive (haute teneur en protéines, en vitamines A et C, en thiamine et en sels minéraux), pour ses bienfaits dans la digestion ainsi que pour son action antiseptique, antivirale, cardio-protectrice, tonique, diurétique... Cependant, de part la forte odeur qui le caractérise, il n'est que peu apprécié, mis à part en faible quantité pour les préparations culinaires.

Aussi, contrairement aux autres plantes qui ont fait l'objet d'extractions les plus diverses : huiles essentielles, concrètes, oléorésines, absolues..., rares sont les publications qui mentionnent des extraits à base d'Allium sativum. Par exemple, JP-04 338 336 décrit des comprimés de traitement de l'obésité par voie orale comprenant un extrait d'ail obtenu à partir d'ail séché soumis à une extraction unique à l'eau ou à l'éthanol. Il s'agit donc d'une oléorésine d'extraction.

La présente invention se propose donc de combler cette lacune en fournissant de nouvelles compositions, en particulier de nouveaux extraits d'ail, solubles dans l'eau, dont les propriétés peuvent être avantageusement exploitées par l'industrie, notamment dans l'industrie pharmaceutique ou dans l'industrie cosmétique, et dont le procédé d'obtention est à la fois facile et peu coûteux à mettre en oeuvre.

L'invention vise également à proposer des utilisations de ces compositions, notamment pour le traitement thérapeutique de l'obésité, ou pour le traitement cosmétique topique de la peau, de la cellulite et/ou des surcharges adipeuses dermiques localisées.

L'invention concerne donc une absolue de bulbes d'Allium sativum.

Une absolue est un extrait résultant d'au moins deux étapes d'extraction. La première étape est effectuée avec un solvant d'extraction volatil non aqueux -notamment non benzénique- et permet d'obtenir une concrète ou une oléorésine. La deuxième étape est effectuée avec l'éthanol à titre de solvant. Il est à noter que les absolues de plantes présentent une odeur très concentrée, caractéristique de la plante. Elles sont habituellement préparées et employées exclusivement dans l'industrie de la parfumerie. Aussi, ne voyait-on pas l'intérêt d'une absolue réalisée à partir d'ail, dont l'odeur se prête si peu à cette catégorie d'extrait. Aussi, jusqu'à ce jour, rien dans les connaissances actuelles ne relate d'absolues obtenues à partir de bulbes d'Allium sativum.

En effet, JP 51 033066, EP 0 464 521, FR 01/03325 décrivent des extraits d'ail, mais aucun ne décrit une absolue de bulbes d'Allium sativum selon l'invention, ni un procédé d'obtention d'une absolue, ni un extrait qui possède les caractéristiques structurelles ou fonctionnelles d'une absolue.

Avantageusement et selon l'invention, il s'agit d'absolues de bulbes d'Allium sativum obtenues à partir d'au moins une concrète (extrait d'ail frais ou décongelé avec un solvant volatil non aqueux -notamment non benzénique-), notamment d'une concrète extraite à l'hexane (dans tout le texte, le terme ACH désigne une absolue obtenue à partir d'une concrète extraite à l'hexane) ou d'une concrète extraite à l'acétate d'éthyle (dans tout le texte, le terme ACAE désigne une absolue obtenue à partir d'une concrète extraite à l'acétate d'éthyle).

Selon un autre mode de réalisation de l'invention, une absolue de bulbes d'Allium sativum est obtenue à partir d'au moins une oléorésine d'extraction (extrait d'ail sec avec un solvant volatil non aqueux notamment non benzénique-), notamment d'une oléorésine d'extraction extraite à l'acétone (dans tout le texte, le terme AOA désigne une absolue obtenue à partir d'une oléorésine extraite à l'acétone).

L'invention concerne également un procédé d'obtention d'un extrait d'Allium sativum correspondant à une absolue selon l'invention.

Un procédé d'obtention d'un extrait d'Allium sativum selon l'invention comprend les étapes suivantes :
- soumission de bulbes d'Allium sativum à une extraction par un solvant volatil non-aqueux,
- évaporation du solvant de façon à obtenir un premier produit d'extraction.

Selon l'invention, ce premier produit d'extraction est ensuite soumis à une extraction subséquente à l'éthanol de façon à obtenir une absolue.

Il convient de noter que lorsque le matériel végétal utilisé est du matériel frais ou décongelé (après avoir été congelé pour sa conservation) le premier produit d'extraction, obtenu après l'étape d'extraction au solvant, est une concrète. Il s'agit d'une oléorésine d'extraction (ou résinoïde) lorsque le matériel végétal est utilisé à l'état sec pour cette même étape d'extraction.

Dans tout le texte, le terme "bulbes" englobe aussi bien des bulbes de la plante (groupes de gousses d'ail) que des gousses d'ail séparées les unes des autres, pelées ou non.

Avantageusement et selon l'invention, le matériel végétal utilisé dans le procédé selon l'invention est du matériel frais ou décongelé (après avoir été congelé pour sa conservation).

Avantageusement et selon l'invention, l'extraction subséquente à l'éthanol, qui permet notamment d'éliminer des cires du premier produit d'extraction, consiste à :
- dissoudre le premier produit d'extraction dans l'éthanol à température ambiante et à récupérer une première solution éthanolique par filtration,
- refroidir la première solution éthanolique à une température de l'ordre de -10°C puis à la filtrer pour récupérer une seconde solution éthanolique,
- distiller l'éthanol de la seconde solution éthanolique pour obtenir au final une absolue.

Avantageusement et selon l'invention, l'étape d'extraction par un solvant volatil non-aqueux est réalisée avec un solvant volatil non-aqueux et non-benzénique.

Avantageusement et selon l'invention, cette extraction est faite avec l'hexane ou l'acétate d'éthyle à titre de solvant, volatil non-aqueux et non-benzénique.

Selon une variante de l'invention, le matériel végétal utilisé dans le procédé selon l'invention est du matériel végétal à l'état sec. Avantageusement et selon l'invention, l'extraction est faite avec l'acétone à titre de solvant volatil non-aqueux et non benzénique.

L'invention s'étend aussi à une composition comprenant au moins une absolue de bulbes d'Allium sativum, d'applications particulièrement intéressantes, notamment à des fins thérapeutiques et/ou cosmétiques.

La composition selon l'invention est fondée sur la découverte très étonnante et inattendue des effets des absolues selon l'invention au niveau de deux mécanismes biologiques du corps humain : la micro-circulation cutanée et la différentiation des préadipocytes en adipocytes.

Au niveau de la micro-circulation cutanée, l'inventeur a montré que les absolues selon l'invention agissent positivement sur la sécrétion de VEGF (« Vascular Endothelial Growth Factor »).

Le VEGF est l'un des plus importants facteurs de régulation positive de l'angiogénèse (Ferrera N., Endocr. Rev. 13:18-32, 1992), vasodilatation (Folkman J. et al., J. Biol. Chem. 267:10931-10934, 1992), perméabilité vasculaire (Keck P.J., Science 246:1309-1312, 1989). Il est spécifique de l'endothélium vasculaire. C'est l'un des facteurs les plus importants de croissance et de survie de l'endothélium. Il est considéré comme étant le facteur pro-angiogénique majeur. Son action est limitée au niveau des cellules endothéliales (Mustenen T. et al., J Cell. Biol. 129:895-898, 1995) et son expression est en parfaite corrélation avec le processus d'angiogénèse associé au processus de réparation.

De ce fait, les absolues de bulbes d'Allium sativum manifestent un grand intérêt dans la fabrication de médicaments, notamment dans le traitement des désordres cutanés où ces dernières peuvent être utilisées avantageusement en tant qu'activateurs de la micro-circulation cutanée. Elles peuvent également participer efficacement, à titre d'agents complémentaires, à tout traitement thérapeutique ou cosmétique (produit de beauté, soin de la peau ...) dans lequel une amélioration de la micro-circulation est souhaitée.

En effet, la peau est fortement vascularisée. C'est au niveau du derme que siège la circulation cutanée : aucun vaisseau ne pénètre dans l'épiderme, dont les besoins métaboliques sont assurés par diffusion à partir du derme papillaire. La circulation cutanée assure l'oxygénation et la nutrition des différentes structures de la peau. En particulier, au niveau des fibroblastes, elle assure la distribution de toutes les matières premières indispensables pour produire les fibres de collagène et d'élastine. Parallèlement, elle draine les déchets issus de leur métabolisme. C'est à la fois au niveau général de l'organisme et au niveau local tissulaire que le flux sanguin délivré aux tissus est réglé.

Les altérations cutanées traduisent des modifications au niveau du système micro-vasculaire dermiques consécutives, dans tous les cas, à une diminution de la quantité de sang délivrée au niveau de la peau, via une atrophie du système micro-circulatoire dermique.

Par ailleurs, il a été également démontré que les absolues selon l'invention peuvent être avantageusement utilisées comme agents actifs anti-adipeux dans des compositions à titre cosmétique, pour le traitement préventif ou curatif de la cellulite et des surcharges adipeuses localisées, ou à titre thérapeutique pour le traitement de l'obésité.

Actuellement, les crèmes amincissantes connues contiennent au moins un agent actif qui favorise la lipolyse et/ou inhibe la lipogénèse, et/ou vise à restaurer la micro-circulation capillaire et lymphatique. Les compositions proposées par l'invention, quant à elles, ouvrent une nouvelle voie dans la lutte contre la cellulite. Tout en agissant sur les cibles classiques, comme les crèmes amincissantes connues, les compositions selon l'invention interviennent au niveau du processus de différenciation des préadipocytes en adipocytes matures, étape clé dans l'expansion de la masse grasse.

De façon schématique, la cellulite correspond à l'accentuation du tissu adipeux dans certaines régions du corps, en particulier sur les hanches, les fesses, les genoux et les avant-bras. Ce tissu adipeux ou masse grasse de l'organisme est présent sous pratiquement toute la surface de la peau. La cellulite est souvent associée à une surcharge adipeuse globale et la stabilité de la masse grasse est un point important dans le contrôle du développement de la cellulite.

Le développement excessif de la masse adipeuse est habituellement lié à une augmentation du volume des adipocytes, et de leur contenu en triglycérides sans qu'il y ait une élévation de leur nombre : on parle d'hypertrophie. Dans certains cas d'excès pondéral, l'hypertrophie s'accompagne d'une hyperplasie, c'est-à-dire d'une augmentation du nombre des cellules adipeuses.

Il est aussi maintenant démontré que le nombre de cellules graisseuses n'est pas déterminé en période périnatale, la formation des adipocytes pouvant s'effectuer tout au long de la vie. Elle se réalise à partir de cellules précurseurs, les préadipocytes. Le préadipocyte est une cellule mince, à morphologie fibroblastigue : c'est lui et non fadipocyte, qui est capable de se multiplier.

Schématiquement, la différenciation terminale adipocytaire se fait en deux étapes avec transformation du préadipocyte en adipocyte mature, ce dernier étant capable d'accumuler des triglycérides.

La présente invention met en évidence des principes actifs, présents dans les absolues de bulbes d'Allium sativum, permettant par application topique d'entraîner une inhibition de la conversion adipocytaire des préadipocytes en adipocytes matures.

Donc, les absolues d'Allium sativum selon l'invention agissent sur un versant nouveau de la lutte contre la cellulite : en limitant le recrutement de préadipocytes dormants ils limitent l'expansion du tissu adipeux.

Mais, ce n'est pas là leur seule efficacité, car il s'avère qu'avec un seul agent actif anti-adipeux selon l'invention, plusieurs clés pour lutter contre la cellulite sont fournies. En effet, un agent anti-adipeux selon l'invention assure aussi une bonne vascularisation du tissu adipeux.

Les absolues selon l'invention constituent donc des agents actifs anti-adipeux d'efficacité maximale rendant inutile de multiplier les combinaisons d'actifs dans les formulations cosmétiques ou thérapeutiques amincissantes.

D'autres expérimentations ont également permis de constater, de façon inattendue, que les agents actifs anti-adipeux selon l'invention sont aussi actifs au niveau d'adipocytes hypertrophiés matures. Ils agissent donc sur les deux processus de croissance adipeuse, hyperplasie et hypertrophie, ce qui permet de les utiliser avec succès dans le domaine thérapeutique pour le traitement de l'obésité. De plus, ces agents actifs anti-adipeux agissent localement sur la cellule adipeuse sans entraîner la libération dans la circulation sanguine d'une partie de ses acides gras qui, faute d'être brûlés, pourraient s'avérer préjudiciables en terme de santé (dépôt sur la paroi artérielle ou le tissu intra-abdominal). Un autre avantage constaté des agents actifs anti-adipeux selon l'invention réside dans le fait que les phénomènes obtenus sont réversibles et que par conséquent ils ne conduisent pas à un blocage du mécanisme physiologique.

L'invention propose ainsi de façon générale, une composition comprenant au moins une absolue selon l'invention.

L'invention s'étend aussi à une composition thérapeutique, notamment pour le traitement topique de désordres cutanés et/ou de l'obésité comprenant au moins une absolue selon l'invention. L'invention s'étend aussi à une composition cosmétique pour le traitement topique préventif ou curatif des désordres de la peau et/ou de la cellulite et des surcharges adipeuses localisées dermiques comprenant au moins une absolue selon l'invention. Par traitement des surcharges adipeuses dermiques localisées, on entend un traitement de la masse graisseuse localisée aussi bien au niveau du derme que de l'hypoderme.

Avantageusement, une composition selon l'invention comprend une quantité d'au moins une absolue selon l'invention adaptée pour au moins sensiblement induire une sécrétion de VEGF et/ou au moins sensiblement inhiber la différenciation des préadipocytes en adipocytes matures, sans sensiblement provoquer d'irritation ou de sensibilisation cutanée.

Avantageusement, la quantité d'absolue(s) selon l'invention est adaptée pour au moins sensiblement favoriser la micro-circulation cutanée et/ou pour le traitement de la cellulite et/ou des surcharges adipeuses localisées. De manière préférée et selon l'invention, cette quantité est comprise entre 3 ppm et 20 ppm -notamment de l'ordre de 10 ppm-.

Il est à noter que dans la pratique de la présente invention, bien que les différentes absolues soient diluées à 25 % dans l'éthanol, la proportion d'absolue(s) en ppm des différentes compositions selon l'invention se réfère quant à elle à une quantité d'absolue(s) "pure(s)", c'est-à-dire une absolue contenant moins de 2 % d'éthanol résiduel.

Selon un mode de réalisation et selon l'invention, la composition comprend au moins une absolue de bulbes d'Allium sativum en solution aqueuse.

Selon l'invention, la composition est constituée d'une solution aqueuse d'au moins une absolue de bulbes d'Allium sativum.

Dans un mode préféré de réalisation, une composition selon l'invention comprend au moins une absolue obtenue à partir d'une concrète extraite de bulbes d'Allium sativum. Avantageusement et selon ce même mode de réalisation, une composition selon l'invention comprend en outre au moins une absolue obtenue à partir d'une oléorésine d'extraction extraite de bulbes d'Allium sativum.

Plus particulièrement, une composition selon l'invention est avantageusement caractérisée en ce qu'elle comprend au moins une absolue de bulbes d'Allium sativum choisie parmi : une absolue obtenue à partir d'une concrète extraite à l'hexane ; une absolue obtenue à partir d'une concrète extraite à l'acétate d'éthyle ; une absolue obtenue à partir d'une oléorésine d'extraction extraite à l'acétone.

Selon l'invention, pour une composition comprenant une absolue obtenue à partir d'une concrète extraite à l'hexane, la quantité de celle-ci est avantageusement comprise entre 3 ppm et 20 ppm -notamment de l'ordre de 10 ppm-.

Selon l'invention, pour une composition comprenant une absolue obtenue à partir d'une concrète extraite à l'acétate d'éthyle ou une absolue obtenue à partir d'une oléorésine d'extraction extraite à l'acétone, la quantité de celle-ci est avantageusement comprise entre 5 ppm et 20 ppm -notamment de l'ordre de 10 ppm-.

Par ailleurs, les compositions thérapeutiques où cosmétiques selon l'invention constituent un soin de la peau, qui de façon préférentielle, est appliqué en traitement externe sur les zones de la peau à traiter au moins une fois par jour, tous les jours de l'année.

Les compositions selon l'invention ne nécessitent aucun autre agent actif que les absolues selon l'invention. Le(les) absolue(s) d'Allium sativum peut(peuvent) constituer le(les) seul(s) agent(s) activateur(s) de la micro-circulation ou agent(s) actif(s) anti-adipeux de ladite composition. Dès lors, une composition selon l'invention peut être exempte d'agent actif complémentaire. En particulier, de préférence l'(les) absolue(s) de bulbes d'Allium sativum est(sont) le(les) seul(s) extrait(s) de plante d'une composition selon l'invention.

Toutefois, une composition selon l'invention peut également être utilisée pour des soins dits "flash" consistant à effectuer un traitement durant 2 ou 3 mois, notamment avant la période estivale. Dans ce cas, elle comprend avantageusement un agent actif complémentaire consistant en un agent lipolytique de tout type connu en soi. Avantageusement une composition selon l'invention comprend au moins un agent lipolytique choisi parmi les agents lipolytiques d'origine naturelle (tel que la caféine et les agents capables de stimuler la synthèse d'AMPc), et les agents lipolytiques de synthèse (composé(s) de bases xanthiques).

D'autres agents actifs complémentaires, notamment à effet cosmétique, peuvent être associés aux absolues selon l'invention.

Une composition selon l'invention peut contenir un certain nombre d'agents capables de : stabiliser le réseau élastique (par exemple des dérivés de silicium) ; et/ou procurer un effet tenseur immédiat (incorporation de polymères de synthèse ou de protéines végétales (soja, germe de blé) ; et/ou assurer une hydratation optimale (incorporation d'agents humectants et/ou d'agents restructurants).

Avantageusement, une composition selon l'invention ne nécessite pas l'incorporation d'agents susceptibles d'améliorer son pouvoir de pénétration (tels que des exfoliants).

La composition selon l'invention peut comprendre tout excipient compatible avec les absolues selon l'invention, acceptable pharmaceutiquement ou cosmétiquement, et pouvant comprendre tous adjuvants appropriés y compris gélifiants, parfums, conservateurs, stabilisants, colorants ... permettant d'obtenir la forme galénique souhaitée et appropriée.

La composition selon l'invention se présente avantageusement sous la forme d'une simple lotion.

L'inventeur a ainsi constaté avec surprise que dans une composition selon l'invention, l'odeur de l'ail peut être plus facilement masquée que celle d'une oléorésine ou d'une concrète issue de cette même plante. Ce résultat était, jusqu'alors, totalement imprévisible pour l'homme du métier, puisque les absolues de plantes sont utilisées principalement pour leur propriété odoriférante. Mais une absolue selon l'invention, outre le fait de présenter des propriétés avantageuses pour le traitement de la cellulite et/ou des surcharges adipeuses et/ou de l'obésité, a également l'avantage de pouvoir entrer dans des compositions de crèmes ou de lotions, de fabrication simple, et d'autoriser un traitement efficace par application topique sans gêne olfactive.

Des exemples de réalisation d'une composition selon l'invention sont donnés ci-après.

Pour la réalisation de lotions à base d'absolue d'ail, les quantités en concentration en volume des divers constituants peuvent être les suivantes :
- Composition n° 1 :
   - absolue de bulbes d'Allium sativum obtenue à partir d'une concrète extraite à l'hexane : 0,0003 % à 0,002 %,
   - excipient : concentration adaptée pour obtenir 100 %,
- Composition n° 2 :
   - absolue de bulbes d'Allium sativum obtenue à partir d'une concrète extraite à l'acétate d'éthyle : 0,0005 % à 0,002 %,
   - excipient : concentration adaptée pour obtenir 100 %,
- Composition n° 3 :
   - absolue de bulbes d'Allium sativum obtenue à partir d'une oléorésine d'extraction extraite à l'acétone : 0,0005 % à 0,002 %,
   - excipient : concentration adaptée pour obtenir 100%.

Une composition selon l'invention est extrêmement simple et peu coûteuse.

L'invention s'étend à l'utilisation pour la préparation d'un médicament pour le traitement thérapeutique et/ou cosmétique, préventif ou curatif des désordres cutanés, de la cellulite, des surcharges adipeuses dermiques localisées ou de l'obésité, caractérisé en ce qu'on applique par voie topique sur des zones de peau à traiter au moins une absolue de bulbes d'Allium sativum à titre d'agent activateur de la micro-circulation et/ou d'agent actif anti-adipeux. L'invention concerne aussi un médicament caractérisé en ce qu'on utilise une composition selon l'invention.

L'invention s'étend aussi à l'utilisation d'au moins une absolue de bulbes d'Allium sativum pour la préparation d'une composition thérapeutique, notamment pour le traitement par voie topique des maladies de la peau ou de l'obésité par application topique sur des zones de peau à traiter, caractérisée en ce qu'on incorpore dans la composition une quantité efficace d'au moins une absolue de bulbes d'Allium sativum à titre d'agent activateur de la micro-circulation et/ou à titre d'agent anti-adipeux.

Avantageusement, dans un procédé ou une utilisation selon l'invention, on utilise une quantité d'au moins une absolue de bulbes d'Allium sativum adaptée pour entraîner au moins sensiblement une sécrétion de VEGF ou une inhibition de la différenciation des préadipocytes en adipocytes matures, et ce sans provoquer d'irritation ni de sensibilisation cutanée.

Avantageusement, dans un procédé ou une utilisation selon l'invention, on applique par voie topique sur au moins une zone de peau à traiter une composition selon l'invention.

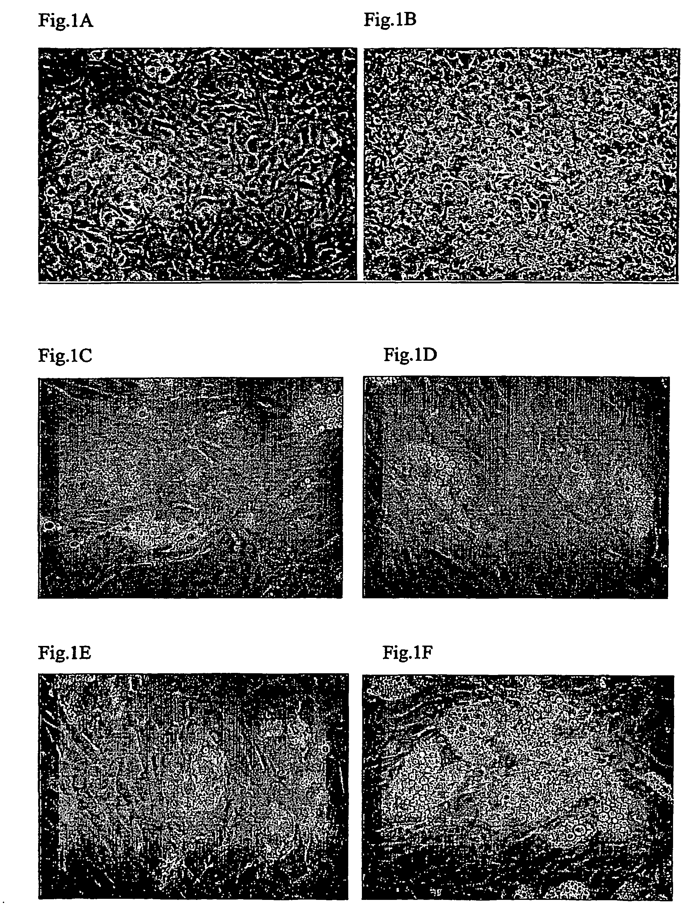

L'efficacité de l'invention ressort des résultats des exemples 1 à 6 décrits ci-après et illustrés en référence aux figures 1A à 1F, 2A et 2B, 3A et 3B, annexées.

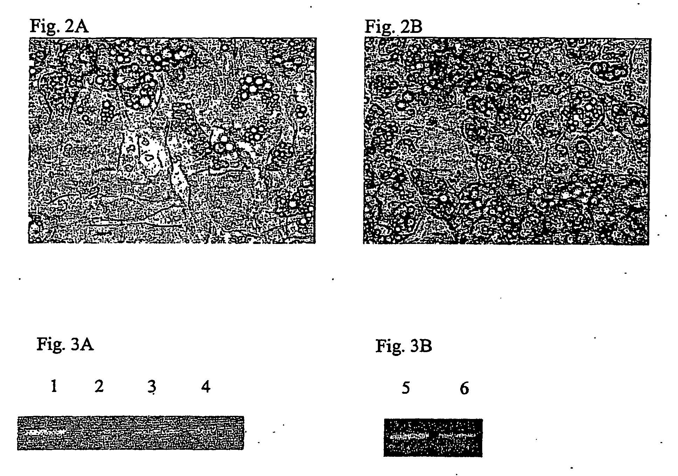

Les figures 1A à 1F, 2A et 2B sont des vues photographiques microscopiques de cellules adipeuses. Les figures 3A et 3B sont des vues photographiques de gels d'électrophorèse.

### EXEMPLE 1 : Obtention des absolues de bulbes d'Allium sativum et préparation des absolues ACH, ACAE et AOA et de solutions les incorporant

Les absolues selon l'invention sont préparées selon un procédé qui en lui-même est classique, bien connu et bien défini dans l'industrie de la parfumerie.

### Obtention d'une absolue ACH

Une première étape consiste à préparer une concrète de bulbes d'Allium sativum. Pour ce faire, des gousses d'ail frais, débarrassées de la peau, sont soumises à un broyage mécanique. Une extraction à l'hexane est alors effectuée. Par chauffage modéré, sans porter le mélange à ébullition, et après totale évaporation de l'hexane, la concrète est récupérée. Celle-ci contient les cires et les produits aromatiques. A température ambiante, elle présente un aspect pâteux, de couleur jaune.

Après dissolution de cette concrète dans l'éthanol par lavages, la solution est filtrée puis glacée à -10°C pour éliminer les cires. Après distillation de l'éthanol, l'absolue ACH est obtenue. Diluée à 25 % dans l'éthanol, elle se présente sous l'aspect d'une solution limpide jaune claire, de forte odeur.

### Obtention de l'absolue ACAE

L'ACAE est préparée selon un protocole comparable à celui de l'obtention de l'ACH, la première étape consistant cette fois en une extraction à l'acétate d'éthyle.

L'absolue ACAE, diluée à 25 % dans l'éthanol se présente sous l'aspect d'une solution limpide marron clair, d'odeur moyenne.

### Obtention de l'absolue AOA

L'AOA est préparée selon un protocole comparable à celui de l'obtention de l'ACH, la première extraction se faisant cette fois à partir de gousses d'ail préalablement soumises à une étape de séchage et avec l'acétone. A l'issue de cette première extraction, une oléorésine d'extraction est obtenue. A température ambiante, elle est sous la forme d'une pâte de couleur marron très foncée. On prépare ensuite l'absolue AOA comme indiqué ci-dessus.

Cette absolue AOA diluée à 25 % dans l'éthanol présente l'aspect d'une solution limpide marron, à très faible odeur.

Un test simple permettant de caractériser les absolues selon l'invention et de les différencier de la plupart des autres extraits à base d'ail (oléorésines, concrètes, huiles essentielles, extraits aqueux) consiste à placer la solution à très basse température, notamment de l'ordre de -20°C. Seules les absolues restent liquides et limpides. Aucun dépôt graisseux n'est observé.

### Préparations de solutions aqueuses d'absolues ACH, ACAE, et AOA

Des solutions aqueuses de ACH, ACAE et AOA sont préparées en diluant, au 1/10 dans de l'eau pour culture cellulaire (Eurobio®, France), des solutions mères de chaque absolue (ACH, ACAE ou AOA) à raison de 18,4 µl d'absolue (ACH, ACAE ou AOA) et de 981,6 µl d'eau pour culture cellulaire (Eurobio®, France).

### EXEMPLE 2 : Effet de ACH, ACAE et AOA sur des primo-cultures de cellules micro-vasculaires dermiques humaines.

Au niveau de la micro-circulation cutanée, les vaisseaux qui sont présents dans le derme, principalement dans le derme papillaire, sont composés d'une couche de cellules endothéliales, d'une paroi musculaire, d'une couche discontinue de péricytes, et d'une membrane basale.

La cellule endothéliale a des fonctions importantes, requises dans l'interface sang-tissus : elle joue un double rôle en contrôlant à la fois l'état de tension du vaisseau, donc le débit sanguin tissulaire, et la structure de la paroi artérielle. Elle a des activités sécrétoires et métaboliques, et c'est au niveau de ces cellules que débute le processus d'angiogénèse, assurant la formation de nouveaux vaisseaux.

### CELLULES ET TRAITEMENTS

Des primo-cultures de cellules micro-vasculaires dermiques humaines (HdMEC à p3, Clonetics®), ont été ensemencées en plaques 6 puits (3500 cellules/puits) et cultivées en milieu EBM complet ("endothelial basal medium"), auquel on ajoute des aliquots fournis par le fournisseur (Clonetics®) : 10 ng/ml EGF recombinant ; 5 µg/ml d'insuline ; 1 µg/ml d'hydrocortisone ; 50 µg/ml gentamicine ; 50 ng/ml amphotéricine B ; et 5% SVF. Les expérimentations ont été mises en oeuvre entre les passages 5 et 7. Les cellules HdMEC sont cultivées dans le milieu, auquel on ajoute au final de 3 à 10 µl d'une solution ACH, ou ACAE, ou AOA par millilitre de milieu de culture. Des cellules HdMEC témoins sont maintenues en culture dans le milieu seul. Le milieu de culture est renouvelé tous les 2 jours.

Les critères d'activité retenus sont :
- stimulation de la différenciation des cellules HdMEC. Les modifications morphologiques sont appréciées par analyse morphologique en microscopie phase inverse couplée à une caméra CCD,
- stimulation de la sécrétion du facteur angiogénique VEGF (« Vascular Endothelial Growth Factor »), par le dosage du VEGF sécrété dans le milieu de culture (« Quantikine Human VEGF Immunoassay », R&D Systems, UK).

### RESULTATS

### Modifications Morphologiques

L'addition de ACH, ou ACAE, ou AOA dans le milieu de culture des cellules HdMEC induit des changements morphologiques : les cellules HdMEC s'allongent, en forme de fuseau, avec des expansions vers les cellules adjacentes. Les cellules HdMEC témoins sont majoritairement sous la forme d'une population homogène de cellules polygonales, avec un noyau central et des bords cellulaires indistincts. Ces réarrangements structuraux conduisent à la formation d'une sorte de réseau capillaire-like.

### Sécrétion de VEGF

L'addition de ACH, ou ACAE, ou AOA dans le milieu de culture des cellules HdMEC induit de façon très significative la sécrétion de VEGF dans le milieu de culture. L'absolue de bulbe d'Allium sativum obtenue à partir d'une concrète extraite à l'hexane (ACH), l'absolue de bulbe d'Allium sativum obtenue à partir d'une concrète extraite à l'acétate d'éthyle (ACAE), l'absolue de bulbe d'Allium sativum obtenue à partir d'une oléorésine d'extraction extraite à l'acétone (AOA), agissent sur les fonctions différenciées des cellules endothéliales, sans en modifier les propriétés prolifératives. Cette modulation des taux de VEGF mène à une augmentation de la micro-vascularisation cutanée.

### EXEMPLE 3 : Effet des solutions de ACH, ACAE ou AOA sur la morphologie adipocytaire (figures 1A à 1F)

### Modèles cellulaires et traitement utilisés

Les mécanismes impliqués dans le processus de différentiation adipocytaire ont été étudiés depuis de nombreuses années *in vitro* (revue générale : Klaus S., BioEssays, 19 :215-223, 1997).

Les activités biologiques de ACH, ACAE et AOA sont mises en évidence à partir de cultures de cellules 3T3-F442A (lignée cellulaire d'origine murine, utilisée pour sa capacité à accumuler des lipides), puis confirmées sur des cultures primaires de préadipocytes humains.

Les cellules sont ensemencées dans des plaques 6 puits (densité d'ensemencement : 15000 cellules/puits) et entretenues jusqu'à confluence en milieu DMEM-10% SVD et antibiotiques. A confluence, les préadipocytes sont alors cultivés en milieu DMEM-10% SVF et insuline. Les cellules traitées sont entretenues dans le milieu de culture auquel on ajoute au final 25 µl de l'une des solutions à tester (ACH, ou ACAE, ou AOA) par millilitre de milieu de culture. Les préadipocytes 3T3-F442A témoins sont cultivés dans le milieu de culture auquel on ajoute 25 µl de solution témoin par millilitre de milieu de culture. Le milieu de culture est renouvelé toutes les 48 heures.

### Primo-cultures de préadipocytes humains

Les préadipocytes humains (ZenBio®, USA), sont cultivés conformément aux instructions du fournisseur. A réception, les préadipocytes humains sont cultivés dans le milieu adéquat fourni, auquel on ajoute au final de 3 µl à 10 µl de l'une des solutions à tester (ACH, ou ACAE, ou AOA) par millilitre de milieu de culture. Les préadipocytes témoins sont maintenus en culture dans le milieu seul. Le milieu de culture est renouvelé toutes les 3 jours.

### RESULTATS

Au terme de 7 jours (cellules 3T3-F442A, figures 1A et 1B) et de 9 jours (préadipocytes humains, figures 1C à 1F) de traitement l'effet des solutions de ACH, ACAE et AOA sur le processus de différenciation a été étudié sur la base de critères morphologiques, par observation en microscopie des cultures traitées et des cultures témoins. Les cellules sont considérées comme différenciées par analyse morphologique en microscopie phase inverse couplée à une caméra CCD, lorsqu'elles acquièrent un contour rond, et que leur cytoplasme est totalement rempli avec des gouttelettes lipidiques. On constate que les solutions de ACH, ACAE ou AOA inhibent la différenciation cellulaire des préadipocytes murins 3T3-F442A (figures 1A et 1B).

Au terme de 7 jours de traitement chronique la solution ACH (figure 1A), induit une diminution significative du nombre d'adipocytes matures développés. La majorité des cellules gardent leur morphologie fibroblastique préadipocytaire avec une très nette inhibition de l'accumulation lipidique. Bien que non illustrés sur les figures, les mêmes résultats sont obtenus avec les solutions de ACAE et de AOA. Les cellules 3T3-F442A témoins (figure 1B), suivent le processus normal de différenciation terminale et acquièrent les caractéristiques morphologiques d'adipocytes matures, avec un cytoplasme rempli de gouttelettes de triglycérides, comme en témoigne la forte réfringence.

On constate aussi que les solutions de ACH, ACAE ou AOA inhibent le processus de différenciation terminale des préadipocytes humains.

Les préadipocytes humains cultivés en présence d'une solution de ACH (figure 1C) gardent une morphologie préadipocytaire : les cellules humaines restent fusiformes avec très peu de gouttelettes lipidiques intra-cytoplasmiques détectables. Les mêmes résultats sont obtenus avec les solutions de ACAE (figure 1D) et de AOA (figure lE). Comparativement, les préadipocytes humains témoins (figure 1F) perdent leur morphologie fibroblastique, avec apparition d'une forme sphérique et l'existence de nombreuses vésicules lipidiques intra-cytoplasmiques identifiables par leur forte réfringence.

Le traitement chronique par l'absolue de bulbes d'Allium sativum obtenue à partir d'une concrète extraite à l'hexane (ACH), l'absolue de bulbes d'Allium sativum obtenue à partir d'une concrète extraite à l'acétate d'éthyle (ACAE), l'absolue de bulbes d'Allium sativum obtenue à partir d'une oléorésine d'extraction extraite à l'acétone (AOA) limite les changements morphologiques et biochimiques caractéristiques de la différenciation adipocytaire. Au contact de ACH, ACAE et AOA les préadipocytes ne sont plus sensibles à l'environnement hormonal qui régule leur métabolisme lipidique et qui permet normalement la conversion des préadipocytes en cellules adipeuses différenciées matures.

### EXEMPLE 4 : -Effet des solutions de ACH, ACAE ou AOA sur la morphologie d'adipocytes hypertrophiés matures (figures 2A et 2B)

A confluence, les préadipocytes 3T3-F442A sont cultivés durant 4 jours en milieu différenciant (DMEM-10% SVF et insuline). Dans ces conditions, les cellules passent au stade de « adipocytes matures » dont le volume et le contenu en triglycérides a été augmenté. Les cellules sont alors traitées comme précédemment.

Comparativement aux adipocytes matures témoins (figure 2B), qui ont une forme volumineuse sphérique et une accumulation importante de vésicules lipidiques intra-cytoplasmiques, les adipocytes matures traités par une solution de ACH (figure 2A), ont une morphologie cellulaire moins arrondie et un contenu en vésicules lipidiques intra-adipocytaires fortement diminué, de sorte que la solution de ACH, selon l'invention s'avère efficace dans le processus de limitation de l'hypertrophie adipocytaire, en inversant le processus de différenciation. Les mêmes résultats sont obtenus avec les solutions de ACAE ou AOA selon l'invention.

### EXEMPLE 5 : Effet des solutions de ACH, ACAE ou AOA sur l'expression de l'ARNm de PPARγ2 (figures 3A et 3B)

La différenciation des adipocytes est une composante importante du développement du tissu adipeux et de l'obésité. Le processus de différenciation adipocytaire est caractérisé *in vitro* par l'induction programmée de différents gènes régulant la lipolyse lipoprotéique, le captage cellule des acides gras, et la synthèse des acides gras et des triglycérides. Schématiquement, ces gènes peuvent être classés comme marqueurs de différenciation de type : très précoces (tel que l'expression de l'ARNm de la lipoprotéine lipase), précoces (tel que l'expression de l'ARNm de PPARγ2) ou tardifs (tel que l'expression de la leptine) (revue générale : Morrison R.F., Farmer S.R, J. Cell. Biochem Suppl. 32/33 : 59-67, 1999). Ainsi, le stade de différenciation des adipocytes peut être établi précisément en étudiant le niveau d'expression des ARNm pertinents (ARNm de PPARγ2) ou par le niveau d'expression de la leptine.

Le processus de différenciation terminale est caractérisé par l'induction de marqueurs de différenciation, précoces et tardifs (revue générale : Morrison R.F, Farmer S.R., J. Cell. Biochem Suppl. 32/33:59-67, 1999).

La famille des récepteurs nucléaires de type PPAR ("Peroxisomal Proliferator-Activated Receptor") est constituée de 3 sous-types : PPARα, PPARδ, PPARγ . PPARα est fortement exprimé dans le foie, il y régule l'expression des gènes impliqués dans le métabolisme lipidique. L'expression de PPARδ est ubiquitaire ; la fonction de ce récepteur reste encore à déterminer. PPARγ existe sous la forme de deux isoformes PPARγ1 et PPARγ2. C'est PPARγ2 qui est fortement exprimé au niveau du tissu adipeux des mammifères. Lorsque PPARγ2 est activé, il induit la transcription de plusieurs gènes adipocytaires codant pour des protéines et des enzymes impliquées dans la création et le maintien du phénotype adipocytaire. Il joue donc un rôle capital dans la différenciation et le métabolisme des cellules adipeuses.

L'effet sur l'expression de l'ARNm de PPARγ2 des solutions de : ACH, ACAE ou AOA a été déterminé au terme de 3 jours (figure 3A, cellules 3T3-F442A) ou de 6 jours (figure 3B, préadipocytes humains) de traitement. Le contenu en ARNm PPARγ2 est analysé par RT-PCR ("Reverse Transcription-Polymerase Chain Reaction"), après avoir préalablement : purifié l'ARN conformément à la procédure du kit RNeasy Total RNA System (Qiagen®), quantifié la teneur en ARN des échantillons par la mesure et le rapport des absorbances à 260 et 280 nm, et vérifié après migration sur gel d'agarose la qualité des ARN obtenus.

Pour l'étape RT, l'ADN complémentaire (ADNc), est synthétisé à partir de 1 µg d'ARN total (3T3-F442A) ou de 0,2 µg d'ARN total (préadipocytes humains). Les amplifications de l'ADNc codant pour PPARγ2 sont obtenues en présence des amorces Sens et Anti-sens adéquates (Genset®, cf. Tableau 1). Les tailles des produits de PCR sont de 307 paires de bases (cellules 3T3-F442A) et de 582 paires de bases (préadipocytes humains). Les contrôles adéquats (étapes de transcription réverse et amplification par PCR) ont été réalisés. Les mélanges de PCR ont été soumis à 22 cycles (cellules 3T3-F442A) ou 35 cycles (préadipocytes humains) d'amplification par dénaturation (2 minutes à 94°C), hybridation (1 minute à 60°C) et élongation (6 minutes à 72°C). Les produits de PCR sont analysés par électrophorèse en gel d'agarose et visualisés au bromure d'éthidium.

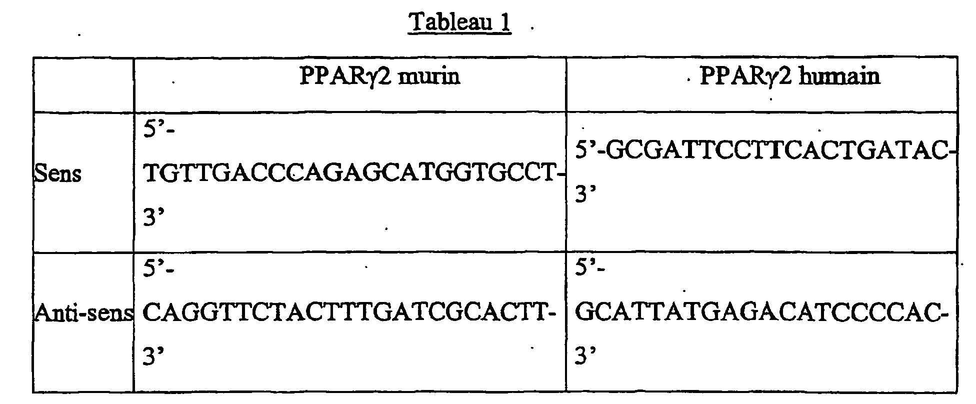

Le niveau d'expression. du facteur de transcription adipogénique PPARγ2 est fortement diminué lorsque les cellules 3T3-F442A ont été au contact d'une solution de ACH (figure 3A, résultat 2), comparativement aux cellules témoins comme en témoigne l'intensité de la bande (figure 3A, résultat 1). Les mêmes résultats sont obtenus avec la solution de ACAE (figure 3A, résultat 3) et la solution de AOA (figure 3A, résultat 4).

De même, les solutions de ACH, ACAE ou AOA selon l'invention inhibent significativement l'expression de l'ARNm PPARγ2 des préadipocytes humains (figure 3B, le résultat 6 illustrant un traitement avec une solution de ACH), comparativement aux préadipocytes humains témoins (figure 3B, résultat 5).

L'absolue de bulbes d'Allium sativum obtenue à partir d'une concrète extraite à l'hexane (ACH), l'absolue de bulbes d'Allium sativum obtenue à partir d'une concrète extraite à l'acétate d'éthyle (ACAE), l'absolue de bulbes d'Allium sativum obtenue à partir d'une oléorésine d'extraction extraite à l'acétone (AOA), agissent au niveau du programme de différenciation en diminuant l'expression du messager de PPARγ2.

### EXEMPLE 6 : Effet des absolues ACH, ACAE et AOA sur la sécrétion de leptine

La capacité des absolues ACH, ACAE et AOA à inhiber le processus de différenciation des préadipocytes humains a été déterminée par la mesure quantitative des taux de leptine sécrétée dans le milieu de culture des différents essais. En effet, la leptine, codée par le gène *ob,* synthétisée et sécrétée par les adipocytes, est considérée comme étant un marqueur tardif du processus de différenciation adipocytaire. *In vitro,* la sécrétion basale de leptine augmente progressivement tout au long du processus de conversion des préadipocytes en adipocytes matures (Mac Dougald, O.A. et al, Proc. Natl Acad Sci, USA, 92:9034-9037, 1995).

L'effet sur la sécrétion de la leptine des absolues ACH, ACAE et AOA a été étudié sur une période de traitement de 9 jours (préadipocytes humains). Les taux de leptine sécrétée par des cellules témoins et par des cellules traitées ont été mesurés dans les différents milieux de culture, prélevés à 3, 6 et 9 jours (jours de renouvellement du milieu). La détermination quantitative des taux de leptine libérée est réalisée par dosage ELISA (R&D Systems Europe), conformément aux instructions du fournisseur. Les résultats sont exprimés en moyenne ± écart type à la moyenne. Les comparaisons statistiques sont réalisées par le test T de Student (p<0,005 représentant le seuil de significativité).

Les résultats sont normalisés comme suit : au terme des 9 jours de traitement, les valeurs en leptine quantifiées pour chacun des traitements au troisième jour, au sixième jour et au neuvième jour, sont cumulées et exprimées en pourcent des valeurs obtenues pour les témoins (Tableau 2).

**Tableau 2 :**

| Effet de ACH, ACAE ou AOA sur la sécrétion de leptine | | | | |
|---|---|---|---|---|
| Traitement | Concentration | Leptine (%) | | Significativité |
| | | Moyenne | Ecart Type à la moyenne | |
| ACH | 3 ppm | 69,4 | 4,4 | P<0,01 |
| ACH | 10 ppm | 36,3 | 0,1 | P<0,001 |
| ACAE | 10 ppm | 81,7 | 2,1 | P<0,01 |
| AOA | 10 ppm | 80,9 | 2,1 | P<0,001 |

Les préadipocytes humains cultivés en présence d'absolue ACH, ACAE et AOA sécrètent dans le milieu de culture des quantités de leptine significativement inférieures à celles détectées dans le milieu de culture des préadipocytes humains témoins non traités. Les résultats obtenus démontrent que les absolues de ACH, ACAE ou AOA agissent au niveau du programme de différenciation et sont capables d'inhiber le processus de conversion adipocytaire.

## Revendications

1. Absolue de bulbes d'Allium sativum.

2. Absolue de bulbes d'Allium sativum obtenue à partir d'au moins une concrète de bulbes d'Allium sativum.

3. Absolue selon la revendication 2, **caractérisée en ce qu'**elle est obtenue à partir d'une concrète extraite à l'hexane.

4. Absolue selon la revendication 2, **caractérisée en ce qu'**elle est obtenue à partir d'une concrète extraite à l'acétate d'éthyle.

5. Absolue de bulbes d'Allium sativum obtenue à partir d'une oléorésine d'extraction de bulbes d'Allium sativum.

6. Absolue de bulbes d'Allium sativum selon la revendication 5, **caractérisée en ce qu'**elle est obtenue à partir d'une oléorésine d'extraction extraite à l'acétone.

7. Composition **caractérisée en ce qu'**elle comprend au moins une absolue de bulbes d'Allium sativum et **en ce qu'**elle est exempte de cires.

8. Composition selon la revendication 7, **caractérisée en ce qu'**elle comprend au moins une absolue de bulbes d'Allium sativum en solution aqueuse.

9. Composition selon la revendication 8, **caractérisée en ce qu'**elle est constituée d'une solution aqueuse d'au moins une absolue de bulbes d'Allium sativum.

10. Composition selon l'une des revendications 7 à 9, **caractérisée en ce qu'**elle comprend une quantité d'au moins une absolue de bulbes d'Allium sativum adaptée pour au moins sensiblement induire une sécrétion de VEGF, sans sensiblement provoquer d'irritation ou de sensibilisation cutanée.

11. Composition selon l'une des revendications 7 à 10, **caractérisée en ce qu'**elle comprend une quantité d'au moins une absolue de bulbes d'Allium sativum adaptée pour au moins sensiblement favoriser la micro-circulation cutanée.

12. Composition selon l'une des revendications 7 à 11, **caractérisée en ce qu'**elle comprend une quantité d'au moins une absolue de bulbes d'Allium sativum adaptée pour au moins sensiblement inhiber la différenciation des préadipocytes en adipocytes matures, sans sensiblement provoquer d'irritation ou de sensibilisation cutanée.

13. Composition selon l'une des revendications 7 à 12, **caractérisée en ce qu'**elle comprend une quantité d'au moins une absolue de bulbes d'Allium sativum adaptée pour le traitement topique de la cellulite et/ou des surcharges adipeuses dermiques localisées.

14. Composition selon l'une des revendications 7 à 13, **caractérisée en ce qu'**elle comprend entre 3 ppm et 20 ppm d'absolue(s) de bulbes d'Allium sativum.

15. Composition selon les revendications 7 ou 14, **caractérisée en ce qu'**elle comprend au moins une absolue obtenue à partir d'une concrète extraite de bulbes d'Allium sativum.

16. Composition selon la revendication 15, **caractérisée en ce qu'**elle comprend en outre au moins une absolue obtenue à partir d'une oléorésine d'extraction extraite de bulbes d'Allium sativum.

17. Composition selon l'une des revendications 7 à 16, **caractérisée en ce qu'**elle comprend au moins une absolue de bulbes d'Allium sativum choisie parmi : une absolue obtenue à partir d'une concrète extraite à l'hexane ; une absolue obtenue à partir d'une concrète extraite a l'acétate d'éthyle ; une absolue obtenue à partir d'une oléorésine d'extraction extraite à l'acétone.

18. Composition thérapeutique selon l'une des revendications 7 à 17.

19. Procédé de traitement cosmétique de la peau, **caractérisé en ce qu'**on applique par voie topique sur au moins une zone de peau à traiter au moins une quantité efficace d'absolue de bulbes d'Allium sativum.

20. Procédé de traitement cosmétique de la peau, **caractérisé en ce qu'**on applique par voie topique sur au moins une zone de peau à traiter une composition selon l'une des revendications 7 à 18.

21. Utilisation d'au moins une absolue de bulbes d'Allium sativum pour la préparation d'une composition thérapeutique pour le traitement par voie topique des maladies de la peau.

22. Utilisation d'au moins une absolue de bulbes d'Allium sativum pour la préparation d'une composition thérapeutique pour le traitement par voie topique de l'obésité.

23. Procédé d'obtention d'un extrait de bulbe d'Allium sativum comprenant les étapes suivantes :
- soumission de bulbes d'Allium sativum à une extraction par un solvant volatil non-aqueux,
- évaporation du solvant de façon à obtenir un premier produit d'extraction,
**caractérisé en ce que** le premier produit d'extraction est soumis à une extraction subséquente à l'éthanol de façon à obtenir une absolue.

24. Procédé selon la revendication 23, **caractérisé en ce que** l'extraction subséquente à l'éthanol consiste à :
- dissoudre le premier produit d'extraction dans l'éthanol à température ambiante et à récupérer une première solution éthanolique par filtration,
- refroidir la première solution éthanolique à une température de l'ordre de -10°C puis à la filtrer pour récupérer une seconde solution éthanolique,
- distiller l'éthanol de la seconde solution éthanolique pour obtenir au final une absolue.

25. Procédé selon l'une des revendications 23 ou 24, **caractérisé en ce que** l'étape d'extraction par un solvant volatil non-aqueux est réalisée au moyen d'un solvant volatil non-aqueux et non-benzénique.

## Patentansprüche

1. Absolutes Öl aus Allium sativum.

2. Absolutes Öl aus Allium sativum, die von wenigstens einem Concrete von Allium sativum erhalten wird.

3. Absolutes Öl nach Anspruch 2, **dadurch gekennzeichnet, dass** es von einem mit Hexan extrahierten Concrete gewonnen wurde.

4. Absolutes Öl nach Anspruch 2, **dadurch gekennzeichnet, dass** es von einem mit Ethylacetat extrahierten Concrete gewonnen wurde.

5. Absolutes Öl aus Allium sativum, das von einem Extraktionsoleoresin von Allium sativum erhalten wurde.

6. Absolutes Öl aus Allium sativum nach Anspruch 5, **dadurch gekennzeichnet, dass** es von einem mit Aceton extrahierten Extraktionsoleoresin erhalten wurde.

7. Zusammensetzung, **dadurch gekennzeichnet, dass** sie wenigstens ein absolutes Öl aus Allium sativum umfasst, und **dadurch**, dass sie wachsfrei ist.

8. Zusammensetzung nach Anspruch 7, **dadurch gekennzeichnet, dass** sie wenigstens ein absolutes Öl aus Allium sativum in wässriger Lösung umfasst.

9. Zusammensetzung nach Anspruch 8, **dadurch gekennzeichnet, dass** sie aus einer wässrigen Lösung von wenigstens einem absoluten Öl aus Allium sativum gebildet ist.

10. Zusammensetzung nach einem der Ansprüche 7 bis 9, **dadurch gekennzeichnet, dass** sie eine Menge von wenigstens einem aboluten Öl aus Allium sativum umfasst, die geeignet ist, um wenigstens im Wesentlichen eine VEGF-Sekretion einzuleiten, im Wesentlichen ohne eine Reizung oder Sensibilisierung der Haut hervorzurufen.

11. Zusammensetzung nach einem der Ansprüche 7 bis 10, **dadurch gekennzeichnet, dass** sie eine Menge von wenigstens einem absoluten Öl aus Allium sativum umfasst, die geeignet ist, um wenigstens im Wesentlichen die Hautmikrozirkulation zu fördern.

12. Zusammensetzung nach einem der Ansprüche 7 bis 11, **dadurch gekennzeichnet, dass** sie eine Menge von wenigstens einem absoluten Öl aus Allium sativum umfasst, die geeignet ist, um wenigstens im Wesentlichen die Differenzierung von Präadipozyten in reifen Adipozyten zu hemmen, im Wesentlichen ohne eine Reizung oder Sensibilisierung der Haut hervorzurufen.

13. Zusammensetzung nach einem der Ansprüche 7 bis 12, **dadurch gekennzeichnet, dass** sie eine Menge von wenigstens einem absoluten Öl aus Allium sativum umfasst, die für eine topische Behandlung von Cellulit und/oder von lokalisierten dermalen adipösen Überlastungen geeignet ist.

14. Zusammensetzung nach einem der Ansprüche 7 bis 13, **dadurch gekennzeichnet, dass** sie zwischen 3 ppm und 20 ppm absolutes Öl aus Allium sativum umfasst.

15. Zusammensetzung nach Anspruch 7 oder 14, **dadurch gekennzeichnet, dass** sie wenigstens ein absolutes Öl umfasst, das von einem Concrete erhalten wurde, das aus Allium sativum extrahiert wurde.

16. Zusammensetzung nach Anspruch 15, **dadurch gekennzeichnet, dass** sie ferner wenigstens ein absolutes Öl umfasst, das von einem Extraktionsoleoresin erhalten wurde, das aus Allium sativum extrahiert wurde.

17. Zusammensetzung nach einem der Ansprüche 7 bis 16, **dadurch gekennzeichnet, dass** sie wenigstens ein absolutes Öl aus Allium sativum umfasst, das ausgewählt ist aus: einem absoluten Öl, das von einem mit Hexan extrahierten Concrete erhalten wurde; einem absoluten Öl, das von einem mit Ethylacetat extrahierten Concrete erhalten wurde; einem absoluten Öl, das von einem mit Aceton extrahierten Extraktionsoleoresin erhalten wurde.

18. Therapeutische Zusammensetzung nach einem der Ansprüche 7 bis 17.

19. Verfahren zur kosmetischen Behandlung der Haut, **dadurch gekennzeichnet, dass** wenigstens eine wirksame Menge absolutes Öl aus Allium sativum auf topischem Weg auf wenigstens eine Zone der zu behandelnden Haut appliziert wird.

20. Verfahren zur kosmetischen Behandlung der Haut, **dadurch gekennzeichnet, dass** eine Zusammensetzung nach einem der Ansprüche 7 bis 18 auf topischem Weg auf wenigstens eine Zone der zu behandelnden Haut appliziert wird.

21. Verwendung von wenigstens einem absoluten Öl aus Allium sativum für die Herstellung einer therapeutischen Zusammensetzung für die Behandlung von Erkrankungen der Haut auf topischem Weg.

22. Verwendung von wenigstens einem absoluten Öl aus Allium sativum für die Herstellung einer therapeutischen Zusammensetzung für die Behandlung von Fettleibigkeit auf topischem Weg.

23. Verfahren zum Erhalten eines Extrakts aus Allium sativum, das die folgenden Schritte umfasst:
- Unterziehen von Allium sativum einer Extraktion mit einem nichtwässrigen flüchtigen Lösungsmittel,
- Verdunstenlassen des Lösungsmittels, um ein erstes Extraktionsprodukt zu gewinnen,
**dadurch gekennzeichnet, dass** das erste Extraktionsprodukt einer Folgeextraktion mit Ethanol unterzogen wird, um ein absolutes Öl zu gewinnen.

24. Verfahren nach Anspruch 23, **dadurch gekennzeichnet, dass** die Folgeextraktion mit Ethanol aus den folgenden Schritten besteht:
- Auflösen des ersten Extraktionsprodukts in dem Ethanol bei Umgebungstemperatur, um eine erste Ethanollösung durch Filtration zurückzugewinnen,
- Abkühlen der ersten Ethanollösung auf eine Temperatur von etwa -10°C und anschließende Filterung, um eine zweite Ethanollösung zurückzugewinnen,
- Destillieren des Ethanols der zweiten Ethanollösung, um schließlich ein absolutes Öl zu gewinnen.

25. Verfahren nach Anspruch 23 oder 24, **dadurch gekennzeichnet, dass** die Extraktionsstufe durch ein nichtwässriges flüchtiges Lösungsmittel mittels eines nichtwässrigen und nichtbenzolischen flüchtigen Lösungsmittels realisiert wird.

## Claims

1. Allium sativum bulb absolute.

2. Allium sativum bulb absolute obtained from at least one concrete of Allium sativum bulbs.

3. Absolute according to claim 2, **characterized in that** it is obtained from a concrete extracted with hexane.

4. Absolute according to claim 2, **characterized in that** it is obtained from a concrete extracted with ethyl acetate.

5. Allium sativum bulb absolute obtained from an oleoresin extract of Allium sativum bulbs.

6. Allium sativum bulb absolute according to claim 5, **characterized in that** it is obtained from an oleoresin extract extracted with acetone.

7. Composition **characterized in that** it contains at least one Allium sativum bulb absolute and **in that** it is free from waxes.

8. Composition according to claim 7, **characterized in that** it contains at least one Allium sativum bulb absolute in aqueous solution.

9. Composition according to claim 8, **characterized in that** it consists of an aqueous solution of at least one Allium sativum bulb absolute.

10. Composition according to one of claims 7 to 9, **characterized in that** it contains a quantity of at least one Allium sativum bulb absolute adjusted so as to at least significantly induce VEGF secretion, without significantly causing cutaneous irritation or sensitization.

11. Composition according to one of claims 7 to 10, **characterized in that** it contains a quantity of at least one Allium sativum bulb absolute adjusted so as to at least significantly promote cutaneous microcirculation.

12. Composition according to one of claims 7 to 11, **characterized in that** it contains a quantity of at least one Allium sativum bulb absolute adjusted so as to at least significantly inhibit the differentiation of preadipocytes to mature adipocytes, without significantly causing cutaneous irritation or sensitization.

13. Composition according to one of claims 7 to 12, **characterized in that** it contains a quantity of at least one Allium sativum bulb absolute adjusted for the topical treatment of cellulite and/or of localized dermal adipose deposits.

14. Composition according to one of claims 7 to 13, **characterized in that** it contains between 3 ppm and 20 ppm of Allium sativum bulb absolute(s).

15. Composition according to claims 7 or 14, **characterized in that** it contains at least one absolute obtained from a concrete extracted from Allium sativum bulbs.

16. Composition according to claim 15, **characterized in that** it also contains at least one absolute obtained from an oleoresin extract extracted from Allium sativum bulbs.

17. Composition according to one of claims 7 to 16, **characterized in that** it contains at least one Allium sativum bulb absolute chosen from: an absolute obtained from a concrete extracted with hexane; an absolute obtained from a concrete extracted with ethyl acetate; an absolute obtained from an oleoresin extract extracted with acetone.

18. Therapeutic composition according to one of claims 7 to 17.

19. Cosmetic skin treatment process **characterized in that** at least one effective quantity of Allium sativum bulb absolute is applied topically to at least one area of skin to be treated.

20. Cosmetic skin treatment process **characterized in that** a composition according to one of claims 7 to 18 is applied topically to at least one area of skin to be treated.

21. Use of at least one Allium sativum bulb absolute for the preparation of a therapeutic composition for the treatment of skin complaints by topical application.

22. Use of at least one Allium sativum bulb absolute for the preparation of a therapeutic composition for the treatment of obesity by topical application.

23. Process for obtaining an extract of Allium sativum bulb comprising the following steps:
- extraction of Allium sativum bulbs with a volatile non-aqueous solvent,
- evaporation of the solvent to obtain a first extraction product,
**characterized in that** the first extraction product undergoes a subsequent extraction with ethanol to obtain an absolute.

24. Process according to claim 23, **characterized in that** the subsequent extraction with ethanol consists in:
- dissolving the first extraction product in ethanol at ambient temperature and recovering a first ethanol solution by filtration,
- cooling the first ethanol solution to a temperature in the order of -10°C and then filtering it to recover a second ethanol solution,
- distilling the ethanol from the second ethanol solution to finally obtain an absolute.

25. Process according to one of claims 23 or 24, **characterized in that** the extraction step with a volatile non-aqueous solvent is performed by means of a volatile non-aqueous and non-aromatic solvent.
